# EUROPEAN PATENT SPECIFICATION

(11) **EP 3 352 741 B1**
(45) Date of publication and mention of the grant of the patent: **19.05.2021**
(21) Application number: 16770492.3
(22) Date of filing: 22.09.2016
(51) Int. Cl.: A61K 31/00, A61K 31/195, A61P 17/16, A61K 31/196, A61P 39/00

(54) **INHIBITORS OF PLASMINOGEN FOR TREATING, REDUCING OR PREVENTING RADIATION-INDUCED INJURIES**
INHIBITOREN VON PLASMINOGEN ZUR BEHANDLUNG, VERMINDERUNG ODER VERHINDERUNG STRAHLUNGSINDUZIERTER VERLETZUNGEN
INHIBITEURS DU PLASMINOGÈNE POUR TRAITER, RÉDUIRE OU PRÉVENIR LES BLESSURES INDUITES PAR RAYONNEMENT

(30) Priority: 22.09.2015 SE 1530138
(43) Date of publication of application: 01.08.2018
(73) Proprietor: Omnio AB, 907 19 Umeå (SE)
(72) Inventor: NY, Tor, 903 36 Umeå (SE); FALLAH, Mahsa, 907 30 Umeå (SE); WILCZYNSKA, Malgorzata, 907 32 Umeå (SE); BLOMQUIST, Michael, 903 54 Umeå (SE); JOHANSSON, Mikael, 903 39 Umeå (SE)
(74) Representative: Zacco Sweden AB
(86) International application number: PCT/EP2016/072610
(87) International publication number: WO 2017/050937

(56) References cited:
- WO-A1-00/37071

## Description

### FIELD OF THE INVENTION

The present invention relates to tranexamic acid and compositions comprising tranexamic acid used to reduce or prevent organ, tissue and cellular damage induced by radiation exposure.

### BACKGROUND

Cancer accounts for about 13% of all deaths, which makes it one of the leading causes of death in the world. Depending on the type, cancers are treated with chemotherapy, radiotherapy or surgery alone or in combination. More than 50% of all cancer patients receive some form of radiotherapy during the course of treatment. However, radiotherapy also have side-effects, such as radiation-induced dermatitis (inflammation of the skin) and mucositis (inflammation of mucous membrane). These side-effects sometimes limit the therapeutic potential and can lead to considerable morbidity. Despite improvement in radiation techniques, many of the irradiated patients experience dermatitis and mucositis. These side-effects impair the quality of life for millions of patients and inflict a burden on the healthcare system.

The deleterious effects of radiation are divided in acute and late effects. Acute effects involve erythema (redness of the skin due to vasodilation), dry- and moist desquamation (shedding of the skin), skin ulcers and necrosis. Late effects involve a poor wound healing, fibrosis, telangiectasia (small, dilated vascular lesions) and carcinogenesis. The molecular mechanisms behind the formation of radiation-induced dermatitis and mucositis are poorly understood. Current treatment of radiation-induced wounds comprises mainly conventional wound treatment with various types of dressings, antibiotics, and corticosteroids. At present, there is no biologically active treatment that significantly improves the healing of radiation-induced dermatitis or mucositis or that reduce the side effects of radiation.

WO00/37071 disclose the use of a synthetic lysine analog, such as tranexamic acid, or a salt thereof, or a combination thereof in the manufacture of a medicament for topical application for the treatment of skin diseases.

### Radiation-induced side-effects and current treatment strategies

More than 50% of all cancer patients receive some form of radiotherapy either as a sole treatment or in connection with surgery or chemotherapy. Although the strategies for radiotherapy are continuously being developed, most patients suffer from radiation-induced side-effects. The side effects are divided into acute effects that appear early after the start of the radiotherapy (erythema, dry desquamation and moist desquamation, skin ulcers and necrosis), and late effects that can be seen more than 3 months after radiotherapy (poor wound healing, fibrosis, telangiectasia, and carcinogenesis). The adverse effects of radiotherapy are dose- and schedule dependent, and they are mostly detected in rapidly proliferating tissues, such as the skin, small blood vessels, gastrointestinal tract and bone marrow. In fact, the skin is wounded to different extent after every form of radiotherapy. Radiation-induced dermatitis is sometimes very painful and is known to effect the quality of life of patients. The strategies that are used today to treat radiation-induced wounds are suboptimal. They include cleansers and moisturizers, dressings, as well as antibiotics and topical corticosteroids. Patients are also recommended to protect the skin from sun exposure and other trauma since the skin's ability to heal is reduced. In rare cases, severe radiation wounds also require skin grafting.

### Molecular mechanisms involved in formation and healing of radiation-induced wounds

The molecular mechanisms leading to radiation-induced dermatitis and mucositis are not well understood. However, these mechanisms include elements involved in traumatic wound healing, as well as elements leading to non-healing chronic wounds. The radiation doses that are used for radiotherapeutic treatments provoke an acute inflammation and activate the coagulation cascade, and later also have effects that suppress normal reparative processes. The main mechanism for radiation-induced injuries is the induction of DNA breaks in rapidly dividing cells. This initiates apoptotic cell death, in particular effecting endothelial cells and fibroblasts. Radiation-induced damage of endothelial cells lead to obstruction of capillary lumen, reduced blood flow, ischemic damage and vascular sclerosis (Denham & Hauer-Jensen, 2002, Radiother. Oncol. 63:129; Dormand et al. 2005, Int. Wound J. 2:112). Radiation-induced fibroblast dysfunction leads to defective collagen deposition and subsequent fibrosis, and radiation damage of epithelial cells suppresses the formation of granulation tissue (Tibbs, 1997, Radiother. Oncol. 42:99). Tissue macrophages, that are relatively resistant to radiation, recognize and remove the apoptotic and necrotic cells by phagocytosis. This phagocytosis induces activation of signaling events within the phagocytic cells. Especially phagocytosis of necrotic cells by macrophages results in activation pro-inflammatory response and stimulates infiltration of blood macrophages and neutrophils. This induces a burst of inflammation that destroys tissue and leads to formation of post-radiation wounds. The outbreak of inflammation is one of the main pathogenic factors for the development of radiation-induced dermatitis (Lorimore et al., 2001, Oncogene, 20:7085).

It has been suggested that the plasminogen activator inhibitor-1 (PAI-1) plays a role in radiation-induced intestinal damage, as genetic deficiency of PAI-1 could be shown to protect against radiation-induced intestinal injury. (Abderrahmani et al. 2012, PLoS ONE, 7(4): e35740) PAI-1 is the primary inhibitor of the plasminogen activators (PA) urokinasetype PA (uPA) and tissue-type PA (tPA). uPA and tPA convert plasminogen to plasmin.

### BRIEF DESCRIPTION OF THE INVENTION

The present inventors have demonstrated that plasminogen deficiency and pharmacological inhibition of plasminogen protects against radiation-induced dermatitis. This is an unexpected finding and contrary to the results of Abderrahmani which point in the direction that increased plasmin activity would be protective against radiation induced injuries.

Accordingly, the present invention provides tranexamic acid, and compositions comprising tranexamic acid, for use in a method of treatment for reducing or preventing radiation-induced injuries by protection of normal, non-cancerous cells from adverse effects of radiation exposure The scope of the present invention is defined in the claims; any other disclosure in the present description is provided for referential purposes, only.

In some embodiments, the tranexamic acid and compositions are for treating a subject having radiation therapy or radiation for example, when administered to a subject having cancer or suspected of developing a malignancy or for uncontrolled cellular growth. Other embodiments disclosed herein concern treating a subject having been exposed to radiation, for example, by accident or by a purposeful act such as a nuclear accident or attack. Other embodiments concern protecting or preventing consequences of radiation exposure in a subject undergoing or having undergone a diagnostic procedure.

The compositions disclosed herein comprises an effective amount of one or more inhibitors of plasminogen or one or more inhibitors of a component of the plasminogen activation pathway. The inhibitor can be a synthetic compound, such as tranexamic acid (TXA), epsilon-aminocaproic acid (EACA), alpha-N-acetyl-L-lysine methyl ester (NALME), trans-aminomethylcyclohexanecarbonyl-L-(O-picolyl)tyrosine-octylamide (YO-2), D-Val-Phe-Lys Chloromethyl Ketone; a naturally occurring inhibitor such as plasminogen activator inhibitor-1 (PAI-1), plasminogen activator inhibitor-2 (PAI-2), alpha-2 antiplasmin, aprotinin (Trasylol®, discreplasminin a plasmin inhibitor isolated from Tityus discrepans scorpion venom, AvKTI a Kunitz-type serine protease inhibitor frommthe spider Araneus ventricosus, Bi-KTI a bumblebee (Bombus ignitus) venom Kunitz-type serine protease inhibitor; or an antibody or an antibody fragment directed to plasminogen or to a component of the plasminogen activation pathway, such as an antibody or an antibody fragment directed to plasminogen, plasmin, tPA, or uPA.

In one aspect, the invention provides a pharmaceutical composition for treating, reducing or preventing radiation-induced injuries by protection of normal, non-cancerous cells from adverse effects of radiation exposure, comprising an inhibitor of plasminogen or an inhibitor of a component of the plasminogen activation pathway. The inhibitor of plasminogen is tranexamic acid. Preferably the radiation-induced injury is radiation-induced dermatitis or radiation-induced mucositis.

In another aspect, the invention provides an inhibitor of plasminogen or an inhibitor of a component of the plasminogen activation pathway for use in treating, reducing or preventing radiation-induced injuries by protection of normal, non-cancerous cells from adverse effects of radiation exposure. The inhibitor of plasminogen is tranexamic acid. Preferably the radiation-induced injury is radiation-induced dermatitis or radiation-induced mucositis.

Also disclosed herein is the use of an inhibitor of plasminogen or an inhibitor of a component of the plasminogen activation pathway for the preparation of a pharmaceutical composition for treating, reducing or preventing radiation-induced injuries. Preferably the inhibitor of plasminogen is tranexamic acid. Preferably the radiation-induced injury is radiation-induced dermatitis or radiation-induced mucositis.

Also disclosed herein is a method for treating, reducing or preventing radiation-induced injuries comprising administering an effective amount of an inhibitor of plasminogen or an inhibitor of a component of the plasminogen activation pathway to a subject in need thereof. Preferably the inhibitor of plasminogen is tranexamic acid. Preferably the radiation-induced injury is radiation-induced dermatitis or radiation-induced mucositis.

It is contemplated herein that a subject that is scheduled to undergo radiation therapy can be treated before, during or after radiation therapy. In addition, a subject having had radiation damage due to exposure can be treated even after adverse effects have occurred in order, for example, to reduce any additional adverse effects that can be a consequence of exposure relative to a control not receiving compositions disclosed herein. Treatments after radiation can be before, during, immediately after or up to several days to a month after exposure or treatment of radiation. In accordance with these embodiments, treatments disclosed herein can be used to protect normal, non-cancerous cells, from radiation exposure.

### LEGENDS TO FIGURES

Figure 1. Representative photos of dorsal skin of irradiated wild-type (WT), plasminogen heterozygous (plg^{+/-}), and plasminogen deficient (plg^{-/-}) mice, and uPA/tPA double-deficient mice at different time points after the radiation. Black arrows show skin ulcer, dashed arrows show desquamation.
Figure 2. Scoring of radiation-induced dermatitis. A comparison of quality of dorsal skin in WT (●), plg ^{+/-} (○), and plg^{-/-} (▼) at different time points after radiation. The scores for the double deficient tPA/uPA mice were always 0 and are not shown for clarity. Scoring system: Normal (0), Erythema (1), Desquamation (2), Open wound (3).
Figure 3. Thickness of epidermis in WT mice and plg^{-/-} mice at different days after radiation.
Figure 4. Quantification of neutrophils (A) and neutrophil extracellular traps (NETs) (using citrullinated Histone 3 as a marker) (B), based on immuno-stained skin sections from WT and plg^{-/-} mice at different days after radiation.
Figure 5. Quantification of macrophages in immuno-stained skin sections from WT and plg^{-/-} mice at different days after radiation.
Figure 6. Plasminogen accumulation in the irradiated skin of WT mice measured by ELISA in extracts prepared from skin. Arrow indicates the day when dermatitis appeared.
Figure 7. Levels of IL-6 (A) and TNF-α (B) in irradiated skin of WT mice (●), and plg^{-/-} mice (○), measured by ELISA. The time point when dermatitis starts to be visible in WT mice is nd marked with red arrow.
Figure 8. mRNA expression levels of factors involved in radiation-induced tissue damage in skin samples form WT mice (●), and plg^{-/-} mice (○) taken at different times after radiation. The time point when dermatitis starts to be visible in WT mice is marked with arrow.
Figure 9. Quantification of plasminogen accumulation at 24h after burn in skin of WT mice, WT mice treated with TXA and in control healthy skin.

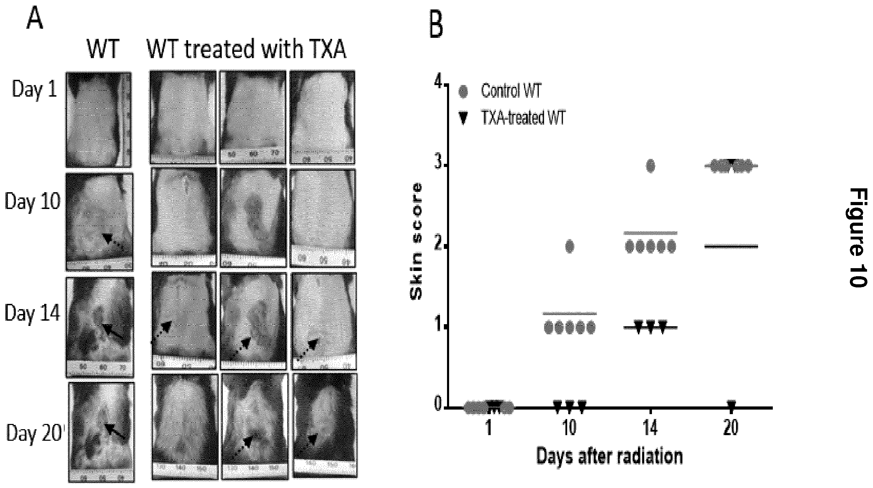

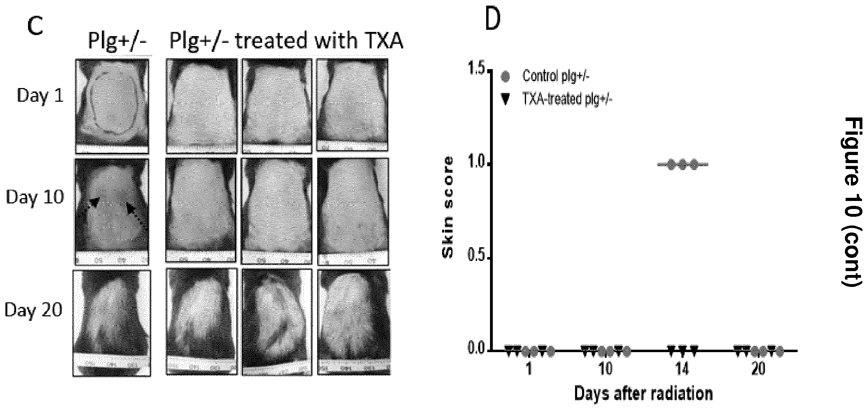

Figure 10. Development of radio-dermatitis in irradiated WT and plg^{+/-} mice that were intraperitonealy treated with TXA. (A) Representative photos of dorsal skin of control irradiated WT mice (●) and irradiated WT mice treated with TXA(▼). (B) A comparison of quality of dorsal skin in WT mice and WT mice treated with TXA. (C) Representative photos of dorsal skin of plg^{+/-} mouse and plg^{+/-} mice treated with TXA. (D) A comparison of quality of dorsal skin in control plg^{+/-} mice (●) and plg^{+/-} mice treated with TXA (▼). Typical irradiated area is marked on photo of control plg^{+/-} taken at day 1. Solid arrows indicate skin ulcers and dashed arrows indicate desquamation.

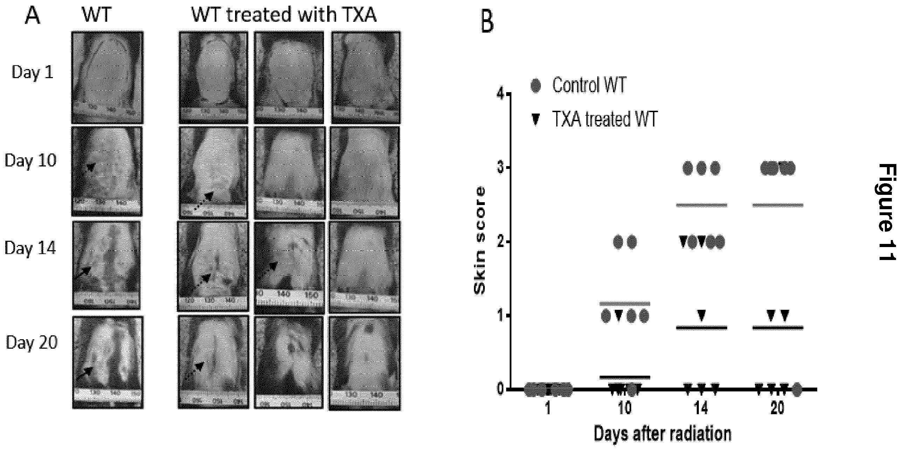

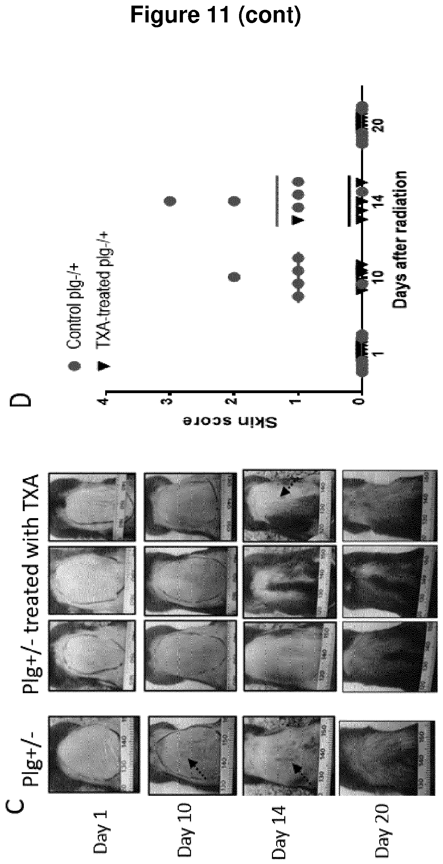

Figure 11. Development of radio-dermatitis in irradiated WT and plg^{+/-} mice that were treated TXA in drinking water. (A) Representative photos of dorsal skin of control WT and WT mice treated with TXA. (B) A comparison of quality of dorsal skin in WT mice (●) and WT mice treated with TXA (▼). (C) Representative photos of dorsal skin of a plg^{+/-} mouse and plg^{+/-} mice treated with TXA. (D) A comparison of quality of dorsal skin in control plg^{+/-} mice (●) and plg^{+/-} mice treated with TXA (▼). Irradiated area is marked on photos taken at day 1. Solid arrows indicate skin ulcers and dashed arrows indicate desquamation.
Figure 12. mRNA expression levels of factors that are known to be involved in radiation-induced tissue damage in skin samples form WT control mice, plg^{-/-} mice, and WT mice treated with TXA in drinking water. Samples were taken at day 9 after radiation.

### DETAILED DESCRIPTION OF THE INVENTION

Radiotherapy (radiation therapy, radiation oncology), can be used alone or as a part of multimodal cancer treatment to control malignant cell growth and/or cellular expansion or abnormal cell growth. Radiation therapy may be prescribed with a curative or palliative treatment intention. Curative treatments are given with the intention to cure the patient and include settings where radiotherapy is given primarily, as an adjuvant treatment to another curative treatment modality such as surgery) or in combination with another treatment modality (such as chemotherapy). Palliative treatment is given to control cancer symptoms and if possible to prolong survival.

Cancer radiotherapy treatments include, but are not limited to, treatment for bladder, breast, kidney, leukemia, skin, lung, myeloma, sarcoma, lymphoma, tongue, prostate, stomach, colon, uterine cancers, melanoma, brain, pancreatic, eye and any other known cancers. In accordance with these embodiments, radiation and/or chemotherapy treatment of a subject for cancer can be accompanied by treatment with a composition disclosed herein. In certain embodiments, radiation-induced damage or side effect, such as radiation-induced dermatitis, radiation-induced intestinal injury, and radiation-induced mucosal injury can be reduced and/or prevented by treatment with a composition disclosed herein.

In another aspect, embodiments disclosed herein provide for tranexamic acid for use in a method of preventing, reducing and treating radiation-induced necrosis and mucosal injury. In certain embodiments, administration of a composition of the invention can be used to protect a subject from radiation-induced mucosal injury. This protection can lead to decreased mortality, improved clinical parameters, and decreased histopathological evidence of necrosis in a subject receiving such a treatment. Further, embodiments disclosed herein can relate to modulation of cellular activities, such as modulation of macrophage activity in a treated subject.

In certain embodiments, tumors are generally known to be more sensitive to photon radiation and can be treated with multiple local doses that cause relatively low damage to normal tissue thus compositions disclosed herein can be used to prevent or treat the low level of damage. In accordance with these embodiments, use of photon radiotherapy during cancer treatment by conventional, three-dimensional conformal, intensity modulated radiotherapy (IMRT) or arc therapies including volumetric arc therapy (VMAT) delivery techniques or other modes has dose-limiting toxicities caused by cumulative effect of radiation and inducing the damage of the stem cells of rapidly renewing normal tissues, for example, bone marrow and gastrointestinal (GI) tract.

### Definitions

### Tranexamic acid (TXA) - Cyclokapron®

CYKLOKAPRON® (tranexamic acid; trans-4 (aminomethyl) cyclohexanecarboxylic acid) is a synthetic analog to lysine. It binds to plasminogen's lysine-binding sites that are located in its kringle domains, and by this prevents plasminogen activation.

As used herein, the term "effective amount" of a composition or agent refers to a quantity of composition or agent sufficient to achieve a desired effect in a subject being treated. An effective amount of a compound can be administered in a single dose or in several doses (daily, for example) during a course of treatment. However, the effective amount of the compound will be dependent on the compound applied, the subject being treated, the severity and type of the affliction, and the manner of administration of the compound.

As used herein, the term "preventing" can refer to inhibiting to the full extent development of something (such as a disease, damage, a condition, etc.), for example, inhibiting the development of cellular or tissue damage after radiation therapy or other exposure to energetic radiation.

As used herein, the term "treating or treatment" refers to a therapeutic intervention that ameliorates a sign or symptom after it has begun to develop.

As used herein, the term "radiation" can refer to energy in the form of waves or moving subatomic particles emitted by an atom or other body as it changes from a higher energy state to a lower energy state. Common sources of radiation include radon gas, cosmic rays from outer space, and medical X-rays. Radiation can be classified as ionizing or non-ionizing radiation, depending on its effect on atomic matter. The most common use of the word "radiation" refers to ionizing radiation. Ionizing radiation has sufficient energy to ionize atoms or molecules, while non-ionizing radiation does not. Radioactive material is a physical material that emits ionizing radiation. There are three common types of radiation: alpha, beta, and gamma radiation. They are all emitted from the nucleus of an unstable atom. X rays produced by diagnostic and metallurgical imaging and security screening equipment are also ionizing radiation, as are neutrons produced by nuclear power generation and nuclear weapons. Sources of radiation exposure include, but are not limited to, radiotherapy, nuclear warfare, nuclear reactor accidents, and improper handling of research or medical radioactive materials.

As used herein, the term "radiation therapy (radiotherapy)" refers to the treatment of a disease (e.g., cancer or another hyperproliferative disease or condition) by exposure of a subject or his/her tissue to radiation or a radioactive substance. Radiotherapy may be used for curative or adjuvant cancer treatment. It is used as palliative treatment where cure is not possible and the aim is for local disease control or symptomatic relief of the subject.

As used herein, the term "cancer" can mean uncontrolled cellular growth, malignant growth or metastatic growth or tumor caused by abnormal and uncontrolled cell division or cellular infiltration or invasion where it can spread to other parts of the body through the lymphatic system or the blood stream.

As used herein, the term "cancer treatment" can mean any treatment for cancer known in the art including, but not limited to, chemotherapy and radiation therapy.

As used herein, the terms "radiodermatitis" (radiation-induced dermatitis) refer to the radiation-induced damage and injuries to the skin seen as acute or chronic continuum of erythema, epilation, desquamation, ulceration, or necrosis occurring as a result of cytokinemediated inflammation and DNA damage.

As used herein, the terms "radiodmucositis" (radiation-induced mucosal damage) refer to the radiation-induced damage and injuries to mucosal tissues.

As used herein, the term "component of the plasminogen activation pathway" refers to plasminogen, plasmin, plasminogen activators exemplified by uPA and tPA.

### EXAMPLES

### EXAMPLE 1

This example demonstrates the mouse model for development of radio-dermatitis and differences in sensitivity to radiation-induced skin damage in mice with different mice genotypes.

### Methods

*Animals:* Plg-heterozygous (plg^{+/-}) mice (Ploplis et al. 1995, Circulation 92:2585) on a C57BL/6 background were intercrossed to generate wild-type (WT), heterozygous (plg^{+/-}), and plg-deficient (plg^{-/-}) mice. The mice were genotyped by a rapid chromogenic assay, as described previously (Ny et al. 1999, Endocrinology 140:5030). Mice deficient in tPA and uPA were backcrossed for 10 generations with C57BL/6 mice (Carmeliet et al. 1994, Nature 368:419). Then, uPA and tPA heterozygous mice were intercrossed to generate the tPA/uPA double-deficient mice. The genotype of these mice was determined by PCR analysis, as previously described (Ny et al. 1997, Eur J Biochem. 244:487). About 8 to 12-week-old mice were used for the experiments. The animals were kept under standard laboratory conditions. The Regional Ethics Committee of Umeå University approved all the experimental protocols.

*Radiation model:* Dorsal skin of mice had been shaved 3 days prior to irradiation. For the irradiation, the mice were anesthetized by intraperitoneal injection of 150 µl mixture containing Ketaminol vet. (Intervet AB, Sollentuna, Sweden) and Dormitor vet. (Orion Pharma AB, Espoo, Finland). Mice were laid into a lead box to protect the whole body from irradiation, while the dorsal skin was gently stretched out through a 4 cm-long gap in the bottom of the box and maintained with medical tape. Irradiation box with a mouse was then placed in Gammacell 40 exactor (Ashford, UK) that has two Caesium-137 sources. Irradiation was given as a single dose of 1 Gy per minute over 15 min (total dose 15 Gy). After the irradiation, mice were observed for the level of consciousness and heart beat for 2 hours and then separately caged.

*Analysis of radiation-induced dermatitis:* Digital photographs of the dorsal skin were taken on the specified days after irradiation. The wound size was quantified using Image J (National Institute of Mental Health, Bethesda, MD). Severity of radio-dermatitis was scored, with the scores defined as: 0 = normal, 1 = erythema, 2 = desquamation, 3 = ulcer.

*Morphological analysis:* Skin from the irradiated area was fixed in 4% paraformaldehyde, embedded in paraffin and sectioned six-micrometer thick and perpendicular to the tissue. The sections were stained with Mayer's hematoxylin (Histolab, Gothenburg, Sweden) and images were taken with a Leica DC300F digital camera attached to a Leica DM LB microscope (Leica, Wetzlar, Germany

### Results

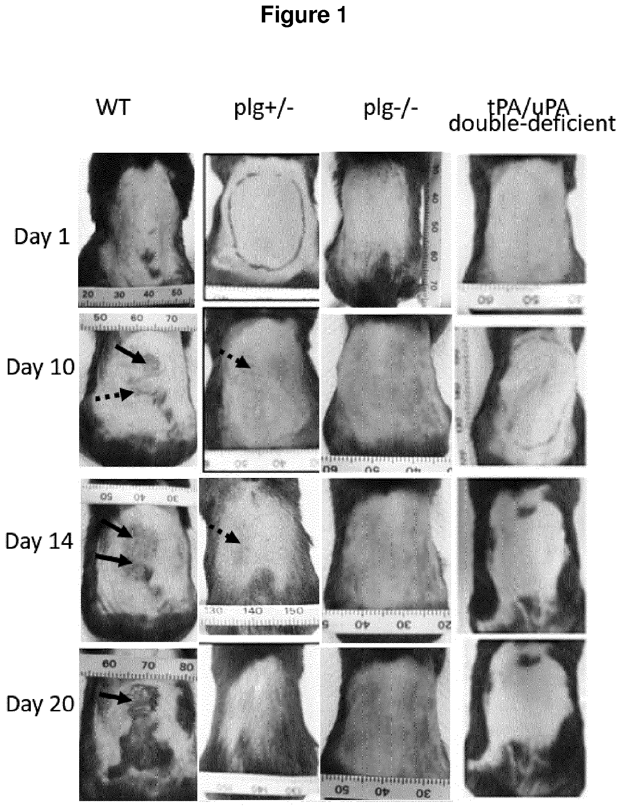

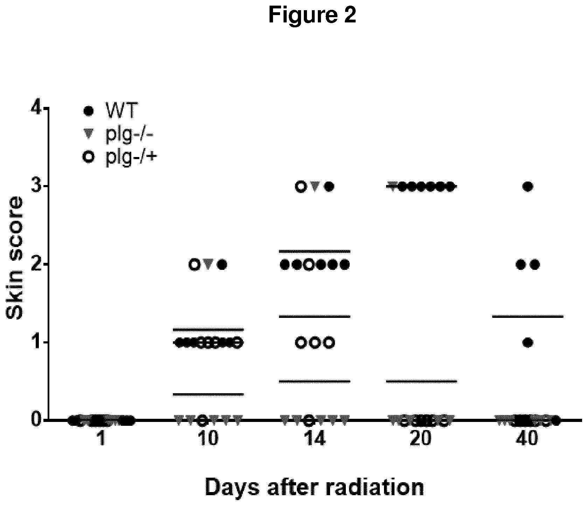

The development of radiodermatitis was monitored by digital photos and scored. Figure 1 shows representative photos of dorsal skin in mice of different genotypes, taken at different days after the irradiation. All WT mice developed erythema at around day 9 after irradiation (data not shown), that converted to a desquamation at day 10 and subsequently to ulcers during days 14 to 20. The plg^{+/-} mice, that contain half of plasminogen level compared to WT mice, developed erythema at about day 10 that healed before day 20 and never converted to a more severe form of radiodermatitis. In contrast, most of plg^{-/-} mice had no clinical signs of dermatitis at any time point after irradiation. Only about 21% of plg^{-/-} mice developed dermatitis which healed before day 20 without converting to ulcers. Importantly, the tPA/uPA double-deficient mice, which have normal plasminogen level but are deficient in plasminogen activators, were resistant to radiodermatitis. The scoring system is shown in Figure 2A. Scoring of the radio-dermatitis in mice (Figure 2B) shows clearly that plg^{+/-} mice developed radio-dermatitis later and less severe than WT mice. These data indicate that the formation of radiation-induced dermatitis is dependent on plasminogen, and that the active form of the molecule, plasmin, is responsible for development of radiation-induced skin damage.

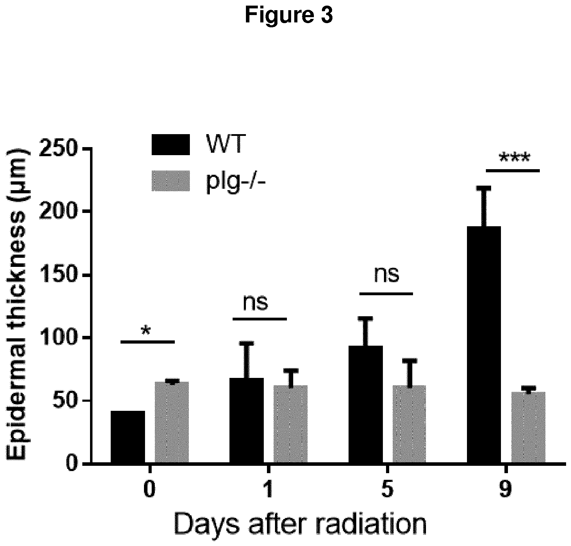

The thickness of epidermis is a measure of skin health and is increased in many pathological conditions (so called skin hyperplasia). Skin sections from irradiated WT and plg^{-/-} mice at different time points after the irradiation were stained with hematoxylin and eosin, and the thickness of epidermis was measured. As shown in Figure 3, the thickness of epidermis in the plg^{-/-} mice was slightly larger than in WT mice before the irradiation (day 0). However, already at day 1 post-irradiation, the thickness of epidermis in WT mice started to increase. At day 9, when erythema was observed, epidermis in WT mice increased about 4.6-fold, as compared to not-irradiated skin. In contrast, the thickness of epidermis was not changed in plg^{-/-} mice following irradiation. This data supports our finding that the presence of plasminogen is obligatory for the induction of pathological changes in skin following irradiation.

### EXAMPLE 2

This example demonstrates that plasminogen accumulates in the irradiated skin and is the key factor that drives inflammation in irradiated skin. This inflammation is the major mechanism for formation of radiation-induced skin damage.

### Methods

In this experiment, mice were irradiated and paraffin section prepared as described in Example 1.

*Immuno-histochemical analyses:* Skin from the irradiated area was fixed, embedded in paraffin and sectioned six-micrometer thick and perpendicular to the tissue. Macrophages were stained with rat anti-mouse F4/80 monoclonal antibody (AbD Serotec, Oxford, UK) and neutrophils were stained with rat-anti mouse Ly-6B.2 monoclonal antibody clone 7/4 (AbD Serotec, Oxford, UK). The primary antibodies were followed by biotinylated goat anti-rat IgG antibodies (Santa Cruz Biotechnology, Dallas, U.S.A) and streptavidin-Alexa Fluor 647 conjugate (ThermoFisher Scientific, Waltham, U.S.A). The NETs (neutrophil extracellular traps) were stained with rabbit anti-citrullinated histone 3 antibody (Abcam, Cambridge, UK) followed by Dylight 488 goat anti-rabbit IgG antibody (Vector Laboratories, Burlingame, U.S.A). DAPI (ThermoFisher Scientific, Waltham, U.S.A) was used for counterstaining and images were taken with a Zeiss Axio Imager Z1 (Zeiss, Oberkochen, Germany).

*Analysis of skin extracts by ELISA:* Dorsal skin samples from irradiated and not-irradiated control mice were homogenized in a lysis buffer (50 mM Tris-HCl buffer pH 8.0 with 120 mM NaCl, 1 mM EDTA, 6 mM EGTA, 1% NP-40 and 1 mM DTT) supplemented with PhosSTOP phosphatase inhibitors and Complete Ultra mini protease inhibitor cocktail tablets (both from Roche, Basel, Switzerland). The skin extracts were then kept at -20°C until use. Total protein concentration in the extracts was quantified using Pierce BCA protein assay kit, according to the manufactural instruction (ThermoFisher Scientific, Waltham, U.S.A). Mouse IL-6 and TNF-alpha levels in the extracts were measured using specific ELISA kits from eBioscience (San Diego, U.S.A). Mouse plasminogen was quantified with a mouse plasminogen-specific ELISA (Omnio AB, Umeå, Sweden).

*Quantitative RT-PCR:* Skin samples were homogenized in TRIzol (Ambion) using Precellys CK28R tubes on a Precellys 24 homogenizer (both from Bertin Technologies, France) according to the manufacturer's instructions. Total RNA was extracted with PureLink RNA Mini Kit (Ambion) according to the manufacturer's instruction. 2.5 µg of total RNA was reverse transcribed using Superscript VILO cDNA synthesis kit (Invitrogen) and diluted 3 fold with DEPC-water. Expression of genes were analyzed using quantitative real-time PCR with Comparative CT method and with TBP mRNA as the internal reference gene. The gene-specific primers and probes (TaqMan Gene Expression Assays) were from Applied Biosytems, and 2x SsoAdvanced Universal Probes Supermix was from BIO-RAD. Each sample was run in triplicate on StepOnePlus Instrument (Applied Biosystems) using real-time PCR cycling conditions for 2x SsoAdvanced Universal Probes Supermix.

### Results

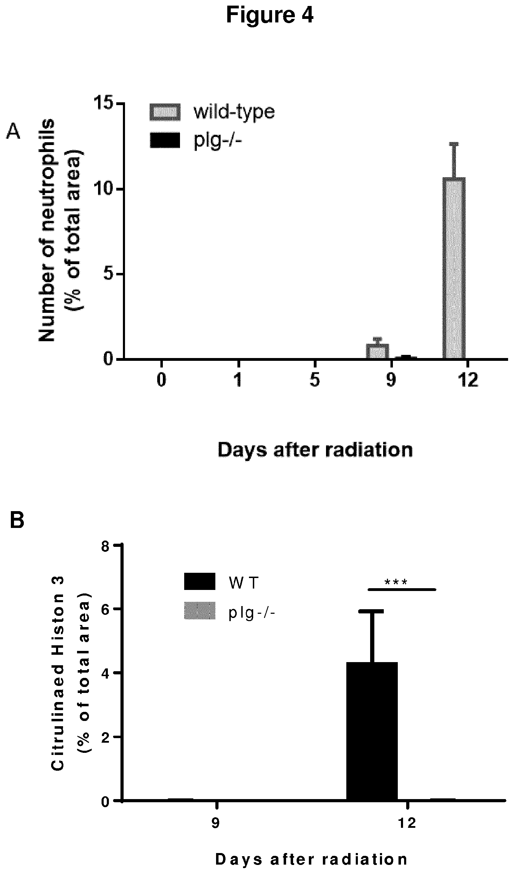

Excessive inflammation is believed to be a major cause for radiation-induced tissue damage (Lorimore et al. 2001, Oncogene 20:7085; Kim et al. 2013 Int. J. Rad. Biol. 89:311). To study the role of plasminogen in this process, skin sections from irradiated WT and plg^{-/-} mice were stained for neutrophils and macrophages. As shown in Figure 4A, there is a significant accumulation of neutrophils at day 9 and day 12 post-irradiation in WT mice. The neutrophil accumulation indicates inflammation and correlates with the development of radio-dermatitis in these mice. However, plg^{-/-} mice have almost no neutrophil at any time points after irradiation.

Neutrophils can form Neutrophil extracellular traps (NETs) via releasing de-condensed chromatin bound with various cytotoxic proteins. The NETs are normally targeting microbes, but can also be formed during inflammation and induce tissue damage (Wong et al. 2015, Nat. Med. 21:815). It was tested whether radiation-induced skin damage may be connected with NETs formation. Skin sections were immuno-stained for citrulinated histon 3 which is a marker for NETs. As shown in Figure 4B, there was no NETs in skin of irradiated plg^{-/-} mice. The NETs were only detectable in WT mice at day 12, when radiodermatitis was already developed. This strongly suggest that NETs formation is not the reason for development of radiodermatitis, but rather a consequence of high inflammation and wound formation.

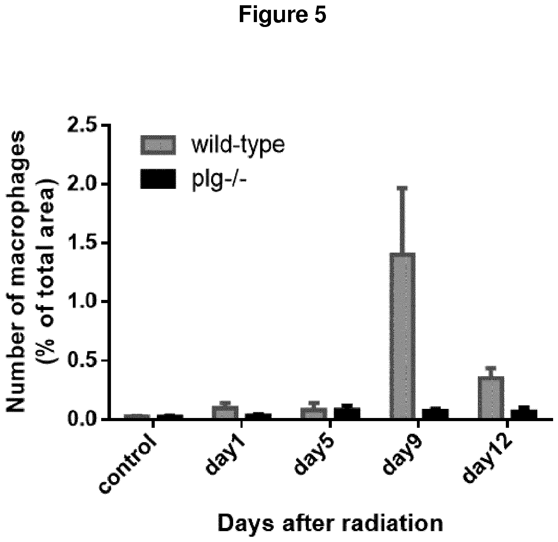

Macrophages started to accumulate in WT mice from day 9 after irradiation, when erythema was visible, but the number of macrophages was lower at day 12 when ulcers were formed (Figure 5). However, there was no accumulation of macrophages in plg^{-/-} mice at these time points.

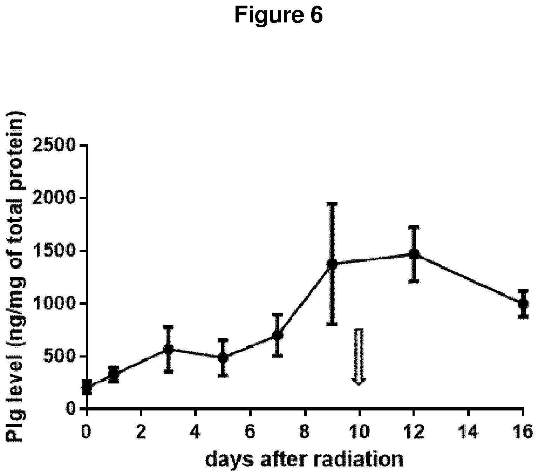

Previously, it has been shown that plasminogen is transported to wounded skin by immune cells, where it activates expression of inflammatory cytokines (Shen et al. 2012, Blood 119:5879). To test whether there is an accumulation of plasminogen after irradiation, skin extracts from WT mice were prepared at different days after irradiation, and quantified the level of plasminogen. As shown in Figure 6, the level of plasminogen in skin of WT mice increased gradually from day 1 after irradiation and reached the highest level at day 9 (about 9-fold of increase).

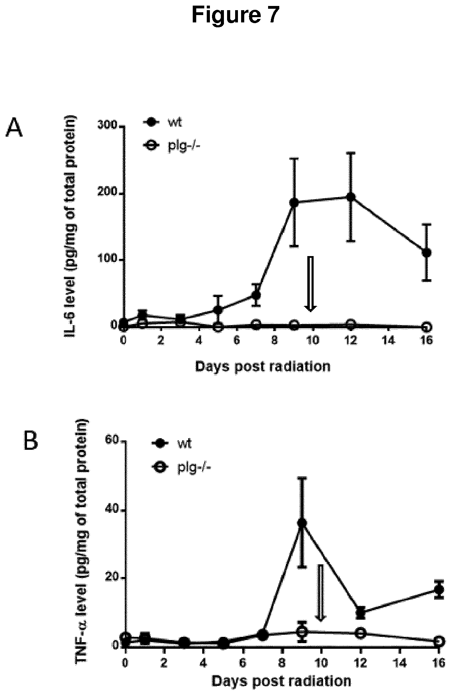

Previous studies have shown that development of acute radiation dermatitis correlates with high levels of various cytokines and chemokines (Kim et al. 2013, Int. J. Rad. Biol. 89:311). As plasminogen is also known to induce expression of pro-inflammatory cytokines (Syrovets & Simmet, 2004, CMLS 61:873; Shen et al. 2012, Blood 119:5879), levels of IL-6 and TNF-α in the skin extracts from WT and plg^{-/-} mice were measured. As shown in Figure 7 in WT mice, the expression of these pro-inflammatory cytokines started to increase from day 5 and reached maximum at day 9 post-irradiation. In contrast, in plg^{-/-} mice, the levels of these cytokines remained on the base level regardless the time point. Therefore, the IL-1β and TNF-α levels in irradiated skin of WT mice start to increase later that plasminogen, but all reach maximum at about the same time (day 9), just when the dermatitis starts.

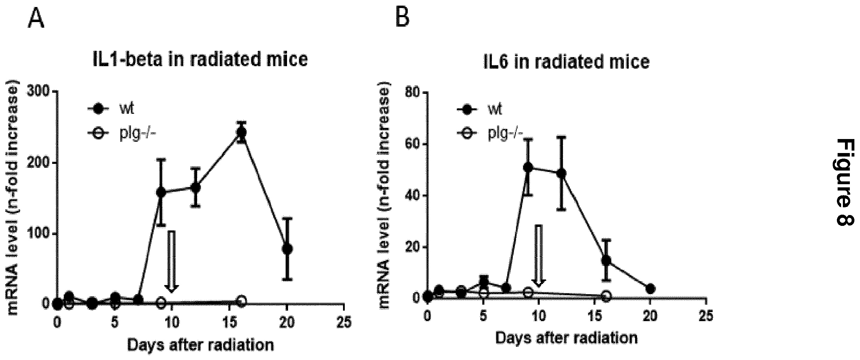

To confirm and extend the data on plasminogen-dependent induction of pro-inflammatory cytokines, RNA from skin extracts were purified and RT-PCR with primers specific for IL-1β and IL-6 were performed. As shown in Figure 8A and B, expression of both these cytokines in WT mice started to increase from day 5 and reach very high levels during days 9-12 when radio-dermatitis was evident. In contrast, expression of these cytokines in plg-deficient mice remained at low levels and was unchanged after irradiation.

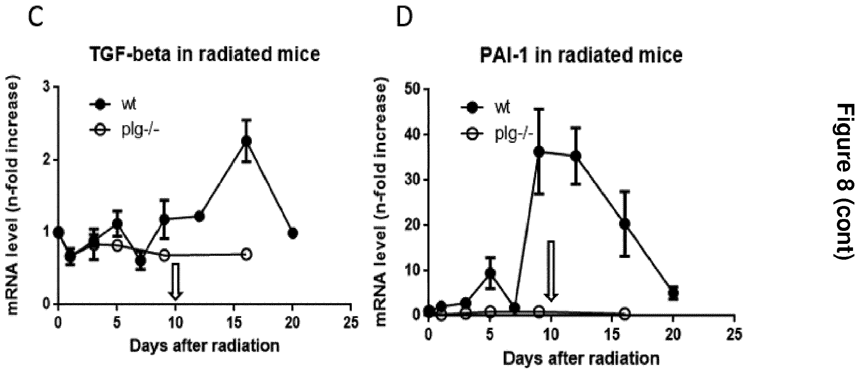

Transforming growth factor, TGF-β, is an important factor that contribute to injury processes after tissue radiation (Kim et al. 2014, Rad. Oncol. J. 32:103). Here it is shown that the expression levels of TGF-β in irradiated skin of WT mice started to increase from day 3 and remained high until day 16. However, no increase in TGF-β levels were detected in plg^{-/-} mice (Figure 8C).

Recently, increased levels of plasminogen activator inhibitor type 1, (PAI-1, serpinE1), were shown to be responsible for radiation-induced blood vessel damage (Milliat et al. 2008, Am J. Path. 172:691). Here it is shown that expression of PAI-1 increases in WT mice from day 3 after irradiation and reaches maximum at day 9. However, the PAI-1 level remained low in all time points in irradiated plg^{-/-} (Figure 8D).

Taken together, these data show that plasminogen accumulates in irradiated skin of WT mice and is the signal that is required for the induction of pro-inflammatory cytokines, as well as TGF-β and PAI-1, which are known to be involved in the molecular mechanisms that are responsible for radiation-induced tissue damage. Therefore, regulating levels and/or activity of plasminogen in irradiated skin/organs may be the key to decrease radiation side-effects in healthy tissues.

### EXAMPLE 3

This example demonstrates that inhibition of plasminogen in WT and plg^{+/-} mice by tranexanic acid (TXA) decreases radiation-induced skin damage. TXA is a lysine analogue that is already used in clinic to prevent excessive bleeding.

### Methods

In this experiment, mice were irradiated as described in Example 1.

*Burn wound:* Mice were anesthetized with Dormicum/Hypnorm and burn wounds were made with a brass stave as described previously (Shen et al. 2012, Blood 119:5879). The mice were given a single injection of Temgesic (Schering-Plough, Brussels, Belgium) the day after burning. All mice were individually caged, and wounds were neither sutured nor dressed.

*Treatment with tranexamic acid (TXA):* WT and plg^{+/-} were intraperitoneally injected with 800 mg/kg of TXA in PBS three times per day. The injections started 2 days before the irradiation and continued for 15 days after the irradiation. Development of dermatitis was documented by digital photos and skin samples were analyzed as described in Example 2.

### Results

As shown in Examples 1 and 2, the development of radiodermatitis depends on the presence of plasminogen, and in fact on its active form, plasmin. It was therefore tested whether the development of radiodermatitis could be decrease or stoped by applying an inhibitor of plasminogen activation. It is known that plasminogen activation *in vivo* occurs on fibrin or on cell surface. Plasminogen binds via kringle domains to carboxyl-terminal lysine residues present on fibrin and plasminogen-specific cell receptors, which leads to a conformational change in plasminogen molecule and allows its activation by a plasminogen activator. The activation of plasminogen can therefore be inhibited by lysine or lysine analogues. Lysine analogues are known to block the binding of plasminogen to cell receptors, and by this, plasminogen activation. Here, TXA which is a lysine analogue used in clinic to treat clotting disorders (Mannucci et al. 2007, N. Engl. J. Med. 356:2301) was applied.

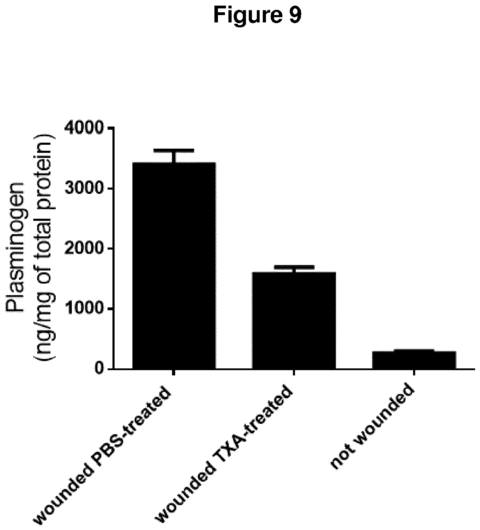

Initially, the burn wound model (Shen et al. 2012, Blood 119:5879) was used to test whether TXA has an effect on plasminogen accumulation in the wound. Burn wounds were introduced to three WT mice and TXA (800 mg/kg) was injected three times at 8h intervals, with the first injection just after wounding. At 24h after wounding, skin samples were collected, and extracts were prepared for determination of plasminogen levels. It was found that the applied dose of TXA was able to inhibit plasminogen accumulation in wounds of WT mice by 50%, when compared to mice treated with PBS (Figure 9).

As TXA decreases accumulation of plasminogen in burn wounds, TXA was injected intraperitoneally to irradiated WT and plg^{+/-} mice. The injections started 2 days before the irradiation and continued for 15 days post-irradiation. As shown in Figure 10A and B, WT mice injected with TXA had delayed development of dermatitis and with a lower severity, as compared to WT mice that receive injections with carrier. When plg^{+/-} mice were treated with TXA, the development of radio-dermatitis was completely blocked (Figure 10C and D).

Taken together, these data demonstrate that the development and severity of radiodermatitis can be ameliorated or stopped by inhibiting of plasminogen activation by using lysine analogues.

### EXAMPLE 4

This example demonstrates that TXA given to mice in drinking water inhibits radiation-induced skin damage.

### Methods

In this experiment, mice were treated as in Example 3, with the exception that TXA was given in drinking water in dose 700mg/kg/day. Real-time PCR was performed as described in Example 3.

### Results

As TXA is often given to patients in tablets, it was tested whether oral administration of TXA to mice could decrease radiodermatitis in these mice.

Oral administration of TXA to WT mice resulted in a significant delay in development of radio-dermatitis and a lower severity of skin damage when compared to control WT mice treated with water only (Figure 11 A and B). Oral administration of TXA to plg^{+/-} mice totally inhibited formation of radiodermatitis (Figure 11 C and D).

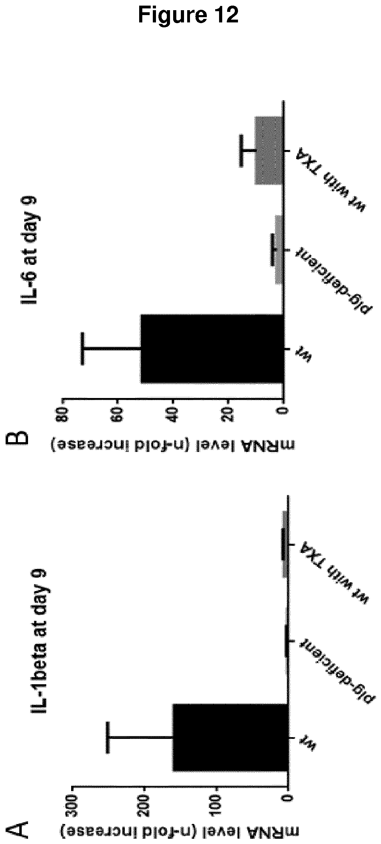

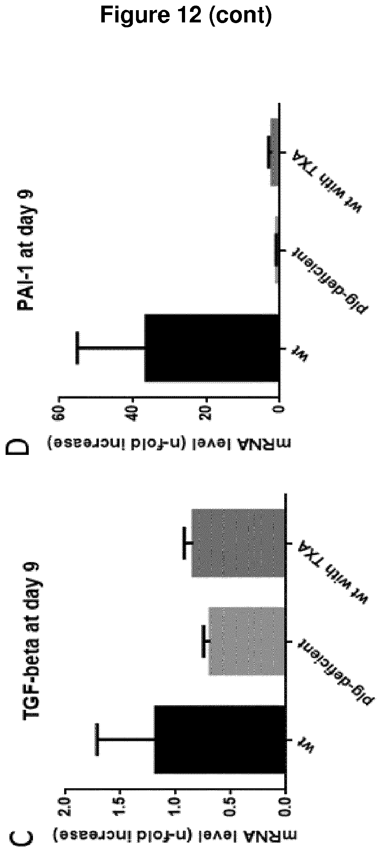

As shown in Example 3, plasminogen was responsible for induction of IL-1beta, IL-6, TGF-β and PAI-1 in irradiated WT mice. Therefore, expression of these proteins in skin of WT mice treated with TXA at day 9 after irradiation were measured. As shown in Figure 12A, the expression of IL-1β is very high in irradiated control WT mice and very low in irradiated plg^{-/-} mice. In the irradiated WT mice that were treated with TXA, the level of IL-1β is also very low. A similar pattern for expression of IL-6, TGF-β and PAI-1 in mice, where TXA decreased expression of these proteins in irradiated WT mice was seen (Figure 12 B, C, and D).

These data demonstrates that oral treatment with TXA inhibits plasminogen's pro-inflammatory effect and, by this, ameliorates or inhibits formation of radiodermatitis.

## Claims

1. Tranexamic acid for use in a method of treatment for reducing or preventing radiation-induced injuries by protection of normal, non-cancerous cells from adverse effects of radiation exposure.

2. Tranexamic acid for use according to claim 1 wherein the radiation-induced injury is selected from radiation-induced dermatitis and radiation-induced mucositis

3. A pharmaceutical composition comprising tranexamic acid for use in reducing or preventing radiation-induced injuries by protection of normal, non-cancerous cells from adverse effects of radiation exposure.

4. The pharmaceutical composition for use according to claim 3 wherein the radiation-induced injury is radiation-induced dermatitis or radiation-induced mucositis.

## Patentansprüche

1. Tranexamsäure zur Verwendung in einem Behandlungsverfahren zur Reduzierung oder Vorbeugung strahleninduzierter Verletzungen durch Schutz von normalen, nicht-kanzerösen Zellen vor Nebenwirkungen der Strahlenbelastung.

2. Tranexamsäure zur Verwendung nach Anspruch 1, wobei die strahleninduzierte Verletzung aus strahleninduzierter Dermatitis und strahleninduzierter Mukositis ausgewählt ist.

3. Pharmazeutische Zusammensetzung umfassend Tranexamsäure zur Verwendung bei der Reduzierung oder Vorbeugung strahleninduzierter Verletzungen durch Schutz von normalen, nicht-kanzerösen Zellen vor Nebenwirkungen der Strahlenbelastung.

4. Pharmazeutische Zusammensetzung zur Verwendung nach Anspruch 3, wobei die strahleninduzierte Verletzung eine strahleninduzierte Dermatitis oder eine strahleninduzierte Mukositis ist.

## Revendications

1. Acide tranexamique destiné à être utilisé dans un procédé de traitement pour réduire ou prévenir des lésions induites par rayonnement par protection de cellules normales et non cancéreuses à partir d'effets indésirables d'exposition aux rayonnements.

2. Acide tranexamique à utiliser selon la revendication 1, dans lequel la lésion induite par rayonnement est choisie parmi la dermatite induite par rayonnement et la mucosité induite par rayonnement

3. Composition pharmaceutique comprenant l'acide tranexamique à utiliser dans la réduction ou la prévention de lésions induites par rayonnement par protection de cellules normales et non cancéreuses à partir d'effets indésirables d'exposition aux rayonnements.

4. Composition pharmaceutique à utiliser selon la revendication 3, dans laquelle la lésion induite par rayonnement est une dermatite induite par rayonnement ou une mucosité induite par rayonnement.
